# EUROPEAN PATENT SPECIFICATION

(11) **EP 2 956 112 B1**
(45) Date of publication and mention of the grant of the patent: **09.01.2019**
(21) Application number: 14751957.3
(22) Date of filing: 13.02.2014
(51) Int. Cl.: A61K 8/21, A61K 8/24, A61Q 11/00

(54) **DENTAL COMPOSITION**
DENTALE ZUSAMMENSETZUNG
COMPOSITION DENTAIRE

(30) Priority: 14.02.2013 US 201313767173
(43) Date of publication of application: 23.12.2015
(73) Proprietor: Karlinsey, Robert, L., Indianapolis, IN 46259 (US)
(72) Inventor: Karlinsey, Robert, L., Indianapolis, IN 46259 (US)
(74) Representative: Barker Brettell LLP
(86) International application number: PCT/US2014/016237
(87) International publication number: WO 2014/127117

(56) References cited:
- US-A1- 2005 084 461
- US-A1- 2007 218 018
- US-A1- 2008 221 681
- US-A1- 2009 324 516
- US-A1- 2010 034 756
- KIYOKO SAKAMOTO ET AL.: "Formation of Fluoridated Hydroxyapatite by Competitive Attack of OH- and F- ions onto alpha- and beta-Tricalcium Bis (Orthophosphate)", JOURNAL OF THE CERAMIC SOCIETY OF JAPAN, vol. 112, no. 1, 2004, pages 6-12, XP002758724,
- WALSH, LJ.: 'Evidence that demands a verdict: latest developments in remineralization therapies.' AUSTRALASIAN DENTAL PRACTICE 2009, pages 49 - 59, XP055276606 Retrieved from the Internet: <URL:http://geriatricdentistry.com/wp/wp-co ntent/uploads/2011/08/L.-Walsh-remin-articl e.pdf>
- SCHEMEHORN, BR ET AL.: 'Comparison of Fluoride Uptake into Tooth Enamel from Two Fluoride Vamishes Containing Different Calcium Phosphate Sources.' THE JOURNAL OF CLINICAL DENTISTRY vol. 22, no. 2, 2011, pages 51 - 54, XP055276608 Retrieved from the Internet: <URL:http://premusa.com/Downloadablefiles/J CD_22_2_Schemehom_et_al.pdf>

## Description

### RELATED APPLICATIONS

This application claims priority to U.S. Patent Application Serial No. 13/767,173, filed on February 14, 2013, which was a continuation-in-part of and claimed priority to utility patent application Ser. No. 11/701,210 filed January 31, 2007 and published as U.S. Patent Pub. No. 2007/0178220; and to U.S. patent application serial no. 12/018627, filed January 23, 2008, which claimed priority to U.S. provisional patent application Ser. No. 60/888,354, filed February 6, 2007, U.S. provisional patent application Ser. No. 60/891,849, filed February 27, 2007, and U.S. provisional patent application Ser. No. 60/941,095, filed May 31, 2007.

### TECHNICAL FIELD

The present novel technology relates generally to the field of chemistry and, more particularly, to a dental remineralizing composition containing calcium and fluoride, which is provided in the form of a homogenous aqueous oral care composition as defined in claim 1.

### BACKGROUND

Tooth mineral is composed predominantly of calcium hydroxyapatite, Ca₁₀(PO₄)₆(OH)₂, which may be partially substituted with anions such as carbonate or fluoride, and cations such as zinc or magnesium. Tooth mineral may also contain non-apatitic mineral phases such as octacalcium phosphate and calcium carbonate.

Tooth loss may occur as a result of dental caries, which is a multifactorial disease where bacterial acids such as lactic acid produce sub-surface demineralisation that does not fully remineralise, resulting in progressive tissue loss and eventually cavity formation. The presence of a plaque biofilm is a prerequisite for dental caries, and acidogenic bacteria such as *Streptococcus mutans* may become pathogenic when levels of easily fermentable carbohydrate, such as sucrose, are elevated for extended periods of time.

Even in the absence of disease, loss of dental hard tissues can occur as a result of acid erosion and/or physical tooth wear; these processes are believed to act synergistically. Exposure of the dental hard tissues to acid causes demineralisation, resulting in surface softening and a decrease in mineral density. Under normal physiological conditions, demineralised tissues self-repair through the remineralising effects of saliva. Saliva is supersaturated with respect to calcium and phosphate, and in healthy individuals saliva secretion serves to wash out the acid challenge, and raises the pH so as to alter the equilibrium in favour of mineral deposition.

Dental erosion (i.e. acid erosion or acid wear) is a surface phenomenon that involves demineralization, and ultimately complete dissolution of the tooth surface by acids that are not of bacterial origin. Most commonly the acid will be of dietary origin, such as citric acid from fruit or carbonated drinks, phosphoric acid from cola drinks and acetic acid such as from vinaigrette. Dental erosion may also be caused by repeated contact with hydrochloric acid (HCl) produced in the stomach, which may enter the oral cavity through an involuntary response such as gastroesophageal reflux, or through an induced response as may be encountered in sufferers of bulimia.

Tooth wear (i.e. physical tooth wear) is caused by attrition and/or abrasion. Attrition occurs when tooth surfaces rub against each other, a form of two-body wear. An often dramatic example is that observed in subjects with bruxism, a grinding habit where the applied forces are high, and is characterised by accelerated wear, particularly on the occlusal surfaces. Abrasion typically occurs as a result of three-body wear and the most common example is that associated with brushing with a toothpaste. In the case of fully mineralised enamel, levels of wear caused by commercially available toothpastes are minimal and of little or no clinical consequence. However, if enamel has been demineralised and softened by exposure to an erosive challenge, the enamel becomes more susceptible to tooth wear. Dentin is much softer than enamel and consequently is more susceptible to wear. Subjects with exposed dentin should avoid the use of highly abrasive toothpastes, such as those based on alumina. Again, softening of dentin by an erosive challenge will increase susceptibility of the tissue to wear.

Dentin is a vital tissue that in vivo is normally covered by enamel or cementum depending on the location i.e. crown versus root respectively. Dentin has a much higher organic content than enamel and its structure is characterised by the presence of fluid-filled tubules that run from the surface of the dentin-enamel or dentin-cementum junction to the odontoblast/pulp interface. It is widely accepted that the origins of dentin hypersensitivity relate to changes in fluid flow in exposed tubules, (the hydrodynamic theory), that result in stimulation of mechanoreceptors thought to be located close to the odontoblast/pulp interface. Not all exposed dentin is sensitive since it is generally covered with a smear layer; an occlusive mixture comprised predominantly of mineral and proteins derived from dentin itself, but also containing organic components from saliva. Over time, the lumen of the tubule may become progressively occluded with mineralised tissue. The formation of reparative dentin in response to trauma or chemical irritation of the pulp is also well documented. Nonetheless, an erosive challenge can remove the smear layer and tubule "plugs" causing outward dentinal fluid flow, making the dentin much more susceptible to external stimuli such as hot, cold and pressure. As previously indicated, an erosive challenge can also make the dentin surface much more susceptible to wear. In addition, dentin hypersensitivity worsens as the diameter of the exposed tubules increases, and since the tubule diameter increases as one proceeds in the direction of the odontoblast/pulp interface, progressive dentin wear can result in an increase in hypersensitivity, especially in cases where dentin wear is rapid.

Loss of the protective enamel layer through erosion and/or acid-mediated wear will expose the underlying dentin, and are therefore primary aetiological factors in the development of dentin hypersensitivity.

It has been claimed that an increased intake of dietary acids, and a move away from formalised meal times, has been accompanied by a rise in the incidence of dental erosion and tooth wear.

In view of this, oral care compositions which can help prevent dental erosion and tooth wear, in addition to dental caries, would be advantageous.

Oral care compositions comprising a source of fluoride ions have been known for many years for combating dental caries. Fluoride ions are known to inhibit plaque bacteria that can cause plaque acid. Fluoride ions are also known to enhance remineralisation and to decrease demineralisation of dental enamel, thereby strengthening dental enamel from acidic challenges.

In more recent years, oral care compositions comprising a source of fluoride ions have also been marketed for combating dental erosion. Some dentifrice compositions have been especially formulated to maximise both the availability of fluoride ions in the composition and their uptake by dental enamel, so to strengthen teeth from both dietary and plaque acidic challenges. It is suggested that such dentifrices suitably do not contain calcium salts.

Attempts have been made over many years to maximise the efficacy of fluoride ions, in strengthening dental enamel, by including a source of calcium and phosphate ions to supplement the natural remineralisation provided by such ions already present in saliva.

However, formulating a source of fluoride ions together with a calcium (phosphate) compound is technically challenging given that the presence of calcium ions together with a source of fluoride ions, can cause the precipitation of insoluble calcium fluoride, thereby significantly reducing the availability of fluoride in an oral care composition. Various solutions to this problem have been suggested including the incorporation of an antinucleating agent or an alkali metal phytate together with the calcium and fluoride ions or the separation of a calcium compound from a source of fluoride ions either by means of a physical barrier in a two phase aqueous composition or by formulating the ingredients in a single phase anhydrous system.

Known anti-carious remineralizing gels, toothpastes and dentifrices comprising from about 0.5 to 10% by weight of α-tricalcium phosphate, tetracalcium phosphate or monocalcium phosphate monohydrate, suitably present in a dry mixture to be reconstituted into a gel by the addition of water which may contain other components including a source of fluoride ions, so to prevent premature reaction of the calcium and fluoride ions.

Some oral care compositions comprise various partially water soluble calcium salts, such as calcium sulphate, together with a source of phosphate and fluoride ions. Such calcium salts are separated until use from the source of phosphate and fluoride ions either by being formulated in a single phase anhydrous system or by means of a physical barrier in a two phase aqueous composition. It is suggested that the oral care compositions preferably contain from about 0.05% to about 15.0% by weight, more preferably from about 0.10% to about 10.0% by weight of the calcium salt(s), from about 0.05% to about 15.0% by weight, more preferably from about 0.10 to about 10.0% by weight of the phosphate salt(s) and from about 0.01% to about 5.0%, more preferably from about 0.02% to about 2.0% by weight of the fluoride salt(s).

Other oral care compositions comprise low quantities (i.e. from 0.01 to 0.09 % by weight of the composition) of various sparingly soluble rod-shaped nanoparticulate calcium compounds such as hydroxyapatite, fluorapatite or calcium fluoride. It is suggested that such compositions can also contain other calcium compounds, which need not be nanoparticulate, such as calcium glycerophosphate, or calcium containing abrasives such as chalk, calcium pyrophosphate or dicalcium phosphate dihydrate. Such compositions can also comprise a source of fluoride ions, preferably in an amount of 0.01 to 0.2% by weight.

There are oral care compositions that comprise nanoparticulate calcium fluoride for combating dental erosion and/or tooth wear. The nanoparticulate calcium fluoride may be present in an amount of 0.001 to 20.0% by weight of the total composition, suitably from 0.01 to 10%, for example from 0.1 to 5.0% by weight of the total composition. It is stated that the oral care composition may further comprise a source of soluble fluoride ions, which can be present in an amount to provide from 25 to 3500 ppm, preferably from 100 to 1500 ppm of fluoride ions.

There are known in the art anti-cariogenic oral hygiene compositions that comprise calcium glycerophosphate and from 0.08 to 7.6% by weight of sodium monofluorophosphate, the sodium monofluorophosphate and the calcium glycerophosphate being present in the composition in a weight ratio of 10:1 to 3:1.

US 2007/218018 describes a sensitivity relief gel which can be used following a dental treatment such as a whitening session. The relief gel contains at least one desensitizing agent, at least one source of fluoride and at least one gelling agent.

Kiyoko Sakamoto et al. "Formation of Fluoridated Hydroxyapatite by Competitive Attack of OH- and F- ions onto α- and β-Tricalcium Bis (Orthophosphate)" Journal of the Ceramic Society of Japan, vol. 112, no. 1, 2004, pages 6-12 describes the examination of the process of formation of fluoridated hydroxyapatite through the hydrolysis of α- or β-tricalcium bis (orthophosphate) in the presence of fluoride ions.

US 2010/034756 describes a solid state method of encouraging the physical interfacing of an organic component to a substantially crystalline inorganic component, including adding predetermined amounts of a substantially crystalline inorganic precursor and a predetermined amount of an organic precursor to yield an admixture, maintaining the admixture in a substantially liquid-free environment, and impacting the precursors together with sufficient energy to fuse the precursors into hybrid compounds.

There remains an electrochemically driven problem with fluoride easily bonding with calcium to form calcium fluoride, effectively removing both calcium and fluoride from bioavailability. The present novel technology addresses this need.

### SUMMARY

Accordingly, in a first aspect, the present novel technology provides a homogenous aqueous oral care composition comprising:
- a fluoride salt that directly provides free fluoride ions in the oral care composition, which provides the composition with from 10ppm to 5000ppm fluoride ions, and
- an unfunctionalised β-tricalcium phosphate,
   wherein the fluoride salt and the β-tricalcium phosphate define a weight ratio of fluoride ions to total calcium ions, and wherein the weight ratio ranges in value from 2:1 to 100:1, such that the β-tricalcium phosphate portion is present in a catalytic and fluoride-stable amount relative to the fluoride salt portion,
   wherein all ingredients of the oral care composition are combined in one mixture, such that they are not kept apart in separate compartments or by means of an anhydrous carrier.

Without wishing to be bound by theory, it is believed that the catalytic amount of unfunctionalised β-tricalcium phosphate can act as a nucleating template or seed, thereby enhancing the efficacy of the fluoride, together with calcium and phosphate ions naturally present in saliva, in remineralising dental enamel.

### DETAILED DESCRIPTION

For the purposes of promoting an understanding of the principles of the novel technology, reference will now be made to the embodiments illustrated in the drawings and specific language will be used to describe the same. It will nevertheless be understood that the scope of the invention is defined by the claims.

As stated above, the present claimed invention provides a homogenous aqueous oral care composition.

The oral care composition comprises water as an excipient and all ingredients of the oral care composition are combined in one mixture; i.e. they are not kept apart in separate compartments or by means of an anhydrous carrier.

The claimed composition includes fluoride salt and β-tricalcium phosphate that define a weight ratio of fluoride ions to total calcium ions ranging from 2:1 to 100:1, such that the β-tricalcium phosphate portion is present in a catalytic and fluoride-stable amount relative to the fluoride salt portion. By "catalytic and fluoride-stable amount" is meant an amount sufficient to enhance the efficacy of fluoride in strengthening dental enamel from acidic challenges but not sufficient to compromise the long term storage stability of fluoride ions in the oral care composition.

In this regard, according to the invention it has now been discovered that unfunctionalised β-tricalcium phosphate is compatible with a source of fluoride ions in an aqueous homogeneous oral care composition, providing the unfunctionalised β-tricalcium phosphate is used in a low enough amount relative to the amount of fluoride ions present.

However, using unfunctionalised β-tricalcium phosphate in such a low amount relative to the source of fluoride ions would not necessarily be expected to augment the natural remineralisation provided by calcium and phosphate ions already present in saliva.

It has further been discovered that such low amounts of β-tricalcium phosphate can enhance the efficacy of fluoride in strengthening dental enamel from acidic challenges. The present novel technology is therefore based upon the two-fold discovery that a fluoride salt can be combined together with unfunctionalised β-tricalcium phosphate in a single phase aqueous oral care composition provided that β-tricalcium phosphate is present in a catalytic and fluoride-stable amount relative to the fluoride salt, whereby the fluoride salt and the β-tricalcium phosphate define a weight ratio of fluoride ions to total calcium ions ranging from 2:1 to 100:1, so as to enhance fluoride efficacy but not to compromise long term storage stability of the fluoride in the composition.

The minimum amount of a β-tricalcium phosphate that can be present relative to the amount of fluoride salt in an oral care composition, to enhance fluoride efficacy, can be determined by adding decreasing amounts of β-tricalcium phosphate to a fixed amount of a fluoride salt and determining the relative efficacy of the oral care compositions (with and without β-tricalcium phosphate) in strengthening dental enamel using a variety of methods, such as surface microhardness measurements as shown in Examples 1 and 2.

The maximum amount of β-tricalcium phosphate that can be present relative to the amount of fluoride salt in an oral care composition, without compromising fluoride stability, can be determined by adding increasing amounts of β-tricalcium phosphate to a fixed amount of a fluoride salt and measuring the levels of available fluoride over time using accelerated aging conditions, as shown in Examples 3 and 4.

The present claimed invention requires that the fluoride salt and the β-tricalcium phosphate define a weight ratio of fluoride ions to total calcium ions, and wherein the weight ratio ranges in value from 2:1 to 100:1, such that the β-tricalcium phosphate portion is present in a catalytic and fluoride-stable amount relative to the fluoride salt portion.

A composition is deemed to have acceptable fluoride stability if the levels of available fluoride are no less than 10% of the theoretical amount that should be present.

The fluoride salts that are used in the claimed invention are those that directly provide free fluoride ions in the oral care composition, such as alkali metal fluorides (eg sodium or potassium fluoride) or stannous fluoride.

Excluded from this definition are salts that indirectly provide free fluoride ions, such as sodium monofluorophosphate, which is hydrolyzed by salivary enzymes in the mouth, releasing free fluoride in situ. Sodium monofluorophosphate has previously been used in aqueous oral care compositions comprising ingredients, such as calcium compounds, which are incompatible with free fluoride ions, especially on long term storage. The present novel technology, however, avoids the need to use such a salt, and instead has found an elegantly simple way of formulating β-tricalcium phosphate with a salt directly providing free fluoride ions.

A preferred fluoride salt is sodium fluoride.

The fluoride salt is present in an amount to provide from 10 to 5000ppm of fluoride ions, eg from 25 to 3500ppm of fluoride ions, preferably from 100 to 1500ppm of fluoride ions, for example the composition may contain 0.1 to 0.5% by weight of sodium fluoride, eg 0.205% by weight (equating to 927ppm of fluoride ions), 0.2542% by weight (equating to 1150ppm of fluoride ions) or 0.315% by weight (equating to 1426ppm of fluoride ions).

By "unfunctionalised β-tricalcium phosphate" is meant β-tricalcium phosphate that is not functionalised in the manner described in the parent applications.

As detailed above, the homogenous aqueous oral care composition of the present claimed invention comprises fluoride salt to provide fluoride ions and an unfunctionalised β-tricalcium phosphate to provide calcium ions. In one embodiment the unfunctionalised β-tricalcium phosphate is used in conjunction with fluorapatite.

Suitable amounts of β-tricalcium phosphate can readily be determined by assessing the fluoride stability and relative fluoride efficacy of the oral care compositions according to the methods described in the Examples.

The present claimed invention requires that the weight ratio of fluoride ions to total calcium ions present in the oral care composition of the present novel technology ranges from 2:1 to 100:1, e.g. the ratio may suitably be from 2.5:1 to 50:1, more suitably from 3:1 to 40:1, preferably from 3.5:1 to 30:1.

By total calcium ions is meant all the calcium ions provided by the calcium compound, both dissolved and undissolved in the oral care composition.

Unlike the aqueous compositions which may comprise solubilised calcium, phosphate and fluoride ions, the compositions of the present novel technology do not require, and suitably do not contain, an alkali metal phytate or an anti-nucleating agent.

Compositions of the present novel technology may further comprise one or more active agents conventionally used in oral healthcare compositions, for example, a desensitising agent, an anti-erosion agent, an anti-plaque agent; an anti-calculus agent, a whitening agent, an oral malodour agent, anti-inflammatory agent, an antioxidant, anti-fungal or wound healing agent or a mixture of at least two thereof. Such agents may be included at levels to provide the desired therapeutic effect.

Compositions of the present novel technology may comprise a desensitising agent, for combating dentin hypersensitivity. Examples of desensitising agents include a tubule blocking agent or a nerve desensitising agent and mixtures thereof. Suitable desensitising agents include a strontium salt such as strontium chloride, strontium acetate or strontium nitrate or a potassium salt such as potassium citrate, potassium chloride, potassium bicarbonate, potassium gluconate and especially potassium nitrate.

A desensitising amount of a potassium salt is generally between 2 to 8% by weight of the total composition, for example 5% by weight of potassium nitrate can be used.

Compositions of the present novel technology may comprise an anti-erosion agent, for example a polymeric mineral surface active agent or a stannous, zinc or copper compound.

Compositions of the present novel technology are aqueous and therefore comprise water. Compositions of the present novel technology may contain appropriate formulating agents such as abrasives, surfactants, thickening agents, humectants, flavouring agents, sweetening agents, opacifying or colouring agents, and preservatives selected from those conventionally used in the oral care composition art for such purposes.

Compositions of the present novel technology are typically formulated in the form of toothpastes, sprays, mouthwashes, or gels.

Compositions of the present novel technology may be prepared by admixing the ingredients in the appropriate relative amounts in any order that is convenient and if necessary adjusting the pH to give a desired value, for example from 5.5 to 9.0

The pH is measured when the composition is slurried with water in a 1:3 weight ratio of the composition to water.

It will be appreciated that the claimed homogenous aqueous oral care composition has utility in the oral care field.

Described but not claimed is a method of combating dental erosion and/or tooth wear which comprises applying an effective amount of a composition as hereinbefore defined to an individual in need thereof.

Described but not claimed is a method of combating dental and/or root caries which comprises applying an effective amount of a composition as hereinbefore defined to an individual in need thereof.

Described but not claimed is a method of combating dentin hypersensitivity which comprises applying an effective amount of a composition as hereinbefore defined to an individual in need thereof.

As detailed above, and as defined in claim 1, the present novel technology relates to an aqueous homogeneous oral care composition comprising a fluoride salt and a catalytic and fluoride-stable amount of β-tricalcium phosphate. The β-tricalcium phosphate is present in an amount sufficient to increase the efficacy of fluoride ions in strengthening dental enamel of teeth against acidic challenges, whilst not significantly compromising the long term storage stability of the fluoride ions in the oral care composition. Such compositions are of use in combating (i.e. helping to prevent, inhibit and/or treat) dental erosion and/or tooth wear. Such compositions are of use in combating dental and/or root caries. Such compositions are of use in combating dentin hypersensitivity.

The novel technology is further illustrated by the following Examples.

### Example 1

### 20-day pH cycling protocol, surface and cross-sectional microhardness and enamel fluoride uptake results - In vitro anti-caries study

White-spot lesions were produced in bovine enamel cores using conventional methods and then organized into the following groups (N=12):
1. Distilled water
2. 1100 ppm F
3. 1100 ppm F + 98 ppm β-TCP

These groups were then subjected to a remineralization/demineralization pH cycling model for 20 days. The model includes two one-minute treatment periods performed an hour apart in the morning, followed by one four-hour lactic acid-polyacrylic acid challenge, and finally two more one-minute treatment periods in the afternoon, administered daily for 10 days. In between the daily treatments and acid challenge, specimens were immersed in artificial saliva (AS). The treatments were diluted three-fold with distilled (DI) water (5 mL treatment solution:10 mL DI water). The treatments and saliva events were magnetically agitated at 300 rpm, while the acid challenge was static. After each treatment and acid challenge, the specimens were rinsed with DI water prior to placement into AS. Four fresh treatment slurries and fresh acid solution were used daily, with the artificial saliva solution changed once daily after the third treatment. Solution used for the acid challenge was the same as used to prepare the initial white-spot lesions.

After 20 days of cycling, the enamel specimens were examined for Vickers surface hardness (200 gf, 15 second dwell time). The change in Vickers hardness number (ΔVHN) was determined as the difference between the post and baseline values (ΔVHN = VHNₚₒₛₜ-VHN_{base}). These data are presented in Table 1.

Then, six enamel specimens were examined for cross-sectional surface microhardnes (CSMH). A series of three indentation lanes per specimen are made under a load of 10 gf at 12.5 µm, 25 gf at 25 and 37.5 µm, and 50 gf at 50, 75, 100, 125 and 150 µm below the specimen surface. The Knoop indentation lengths were then converted to Knoop Hardness Numbers (KHN). Relative to KHN of sound enamel, relative lesions sizes in units of square root of KHN (√KHN) times enamel depth (µm) were then calculated using Simpson's Composite Rule. These data are presented in Table 2.

Enamel fluoride uptake (EFU) was determined for the remaining six specimens in units of micrograms of fluoride per unit enamel area (µg F/cm²). The enamel specimens were immersed and continuously agitated in 0.5 ml of 1 N HClO₄ for 15 seconds. To determine fluoride concentration, 0.25 ml of this etchant was combined with 0.25 ml of 1 N NaOH and 0.5 ml of TISAB II. This solution was magnetically stirred, and a fluoride ion specific electrode was used to measure the free fluoride ion potential. These measurements were then converted to fluoride concentrations by using a fluoride electrode calibration curve constructed from known standards prepared with NaF. These data are presented in Table 3.

The microhardness and enamel fluoride uptake data were analyzed for normality using the Kolmogorov-Smirnov test with p=0.05. One-way analysis of variance (ANOVA) was performed to test for significant differences, followed by comparison tests at a 95% confidence level (p<0.05).

In summary, the addition of a low level of β-TCP to NaF solutions produced improved surface and subsurface benefits in white-spot enamel lesions. Additionally, remineralization benefits from this novel technology may be associated with or without increases in fluoride uptake.

### Surface Microhardness Results:

**Table 1. Summary of surface microhardness results after cycling through the 20-day cycling model. VHN⁰ = mean baseline Vickers Hardness Number (VHN) and (SEM) (N=12); VHN²⁰ = mean VHN (SEM) (N=12) after 20 days of cycling; ΔVHN²⁰ = difference between mean VHN²⁰ (SEM) (N=12) and VHN⁰ after 20 days of cycling.**

| **Group** | **VHN⁰** | **VHN²⁰** | **ΔVHN²⁰** |
|---|---|---|---|
| Distilled Water | 35.8 (2.1) | 35.8 (2.4) | 0.0 (1.6) |
| 1100 ppm F | 35.6 (2.0) | 115.7 (3.2) | 84.2 (3.8) |
| 1100 ppm F + 98 ppm β-TCP | 35.6 (2.0) | 165.0 (3.2) | 128.9 (3.2) |

### CSMH Results:

**Table 2. Summary of cross-sectional microhardness (CSMH) after cycling through the 20-day remin/demin model. ΔZ (√KHN•µm) = mean (SEM) lesion size (N=6) after 20 days of cycling.**

| Group | ΔZ (√KHN•µm) |
|---|---|
| Distilled Water | 539.0 (27.4) |
| 1100 ppm F | 171.8 (24.6) |
| 1100 ppm F + 98 ppm β-TCP | 58.6 (23.5) |

### EFU Results:

**Table 3. Summary of mean (SEM) enamel fluoride uptake (EFU) results from white-spot lesions (N=6) after cycling.**

| **Group** | **EFU (µg F/cm²)** |
|---|---|
| Distilled Water | 0.4 (0.0) |
| 1100 ppm F | 2.9 (0.3) |
| 1100 ppm F + 98 ppm β-TCP | 2.2 (0.3) |

### Example 2

### 20-day pH cycling protocol, surface microhardness and enamel fluoride uptake results - In vitro anti-erosion study

Erosive lesions were produced in bovine enamel cores using 1% citric acid (pH = 3.8) for 30 minutes and then organized into the following groups (N=12):
1. Distilled water
2. 1100 ppm F
3. 1100 ppm F + 186 ppm β-TCP

These groups were then subjected to a remineralization/demineralization pH cycling model for 20 days. This model includes three two-minute treatment periods and five two-minute acid challenge periods. In between these events, the specimens are immersed in artificial saliva (AS). The treatments were diluted three-fold with distilled (DI) water (5 mL treatment solution:10 mL DI water). The treatment and AS systems were magnetically agitated at 300 rpm, while the acid challenge (0.3% citric acid, pH =3.8) was static. After each treatment and acid challenge, the specimens were rinsed with DI water prior to placement into the saliva mixture, which was changed once daily after the third acid challenge.

After 20 days of cycling, the enamel specimens were examined for Vickers surface hardness (200 gf, 15 second dwell time). The change in Vickers hardness number (ΔVHN) was determined as the difference between the post and baseline values (ΔVHN = VHNₚₒₛₜ-VHN_{base}). These data are presented in Table 4.

Enamel fluoride uptake was determined in units of micrograms of fluoride per unit enamel area (µg F/cm²). Six enamel specimens were immersed and continuously agitated in 0.5 ml of 1 N HClO₄ for 15 seconds. To determine fluoride concentration, 0.25 ml of this etchant was combined with 0.25 ml of 1 N NaOH and 0.5 ml of TISAB II. This solution was magnetically stirred, and a fluoride ion specific electrode was used to measure the free fluoride ion potential. These measurements were converted to fluoride concentrations by using a fluoride electrode calibration curve constructed from known standards prepared with NaF. These data are presented in Table 5.

The microhardness and enamel fluoride uptake data were analyzed for normality using the Kolmogorov-Smirnov test with p=0.05. One-way analysis of variance (ANOVA) was performed to test for significant differences, followed by comparison tests at a 95% confidence level (p<0.05).

### Surface Microhardness Results:

**Table 4. Summary of surface microhardness results after cycling through an anti-erosion remin/demin model lasting 20 days. VHN⁰ = mean baseline Vickers Hardness Number (VHN) and (SEM) (N=12); VHN²⁰ = mean VHN (SEM) (N=12) after 20 days of cycling; ΔVHN²⁰ = difference between mean VHN²⁰ (SEM) (N=12) and VHN⁰ after 20 days of cycling.**

| **Group** | **VHN⁰** | **VHN²⁰** | **ΔVHN²⁰** |
|---|---|---|---|
| Distilled Water | 214.6 (2.6) | 264.2 (3.3) | 49.2 (4.4) |
| 1100 ppm F | 214.5 (2.6) | 281.5 (3.7) | 67.7 (2.8) |
| 1100 ppm F + 186 ppm β-TCP | 214.7 (2.5) | 303.9 (3.4) | 89.3 (4.2) |

### Enamel Fluoride Uptake Results:

**Table 5. Summary of mean (SEM) enamel fluoride uptake (EFU) results from eroded enamel (N=6) after cycling through an anti-erosion remin/demin model lasting 20 days.**

| **Group** | **EFU (µg F/cm²)** |
|---|---|
| Distilled Water | 2.7 (0.2) |
| 1100 ppm F | 3.2 (0.1) |
| 1100 ppm F + 186 ppm β-TCP | 3.7 (0.0) |

In summary, the addition of a low level of β-TCP to NaF solutions produced improved surface benefits, including microhardness and fluoride uptake in eroded enamel.

### Example 3 Fluoride Stability

Fluoride availability was measured in triplicate for fluoride plus β-TCP. Solutions/suspensions of 0.24% NaF (1100 ppm F⁻) plus β-TCP were prepared and stored at elevated conditions for 32 days at 40°C. Measurements were made using a fluoride-sensitive electrode and Accumet AR Dual Channel pH meter. TISAB II ionic strength adjuster was added in a 1:1 ratio with a sample volume (e.g. 5 ml), and standards were and measured in order to generate the calibration curve. Conversions from mV to ppm were determined based on the calibration curve and the data are presented in Table 6.

**Table 6. Fluoride compatibility after accelerated aging conditions for 32 days at 40°C in aqueous solutions of sodium fluoride.**

| **System** | **% NaF** | **Theoretical [F⁻]** | **Measured [F⁻] (Mean ± SD)** | **% of 1100 ppm F Control** |
|---|---|---|---|---|
| Distilled Water | 0.0 | 0 ppm | 0.2 ± 0.0 ppm | 0.0 % |
| 1100 ppm F | 0.24% | 1100 ppm | 1101.9 ± 0.0 ppm | 100.0 % |
| 1100 ppm F + 98 ppm β-TCP | 0.24% | 1100 ppm | 1077.6 ± 2.4 ppm | 97.8 % |
| 1100 ppm F + 186 ppm β-TCP | 0.24% | 1100 ppm | 1089.0 ± 7.4 ppm | 98.8 % |

### Example 4

### 30-, 60- and 90-day Fluoride Stability with Low Levels of CaP Systems

### Protocol:

Fluoride availability was measured in triplicate for the fluoride plus β-TCP combinations. Solutions/suspensions of 0.05% NaF (225 ppm F⁻) plus β-TCP were prepared and stored at elevated conditions for 30, 60 and 90 days at 40°C. Measurements were made using a fluoride-sensitive electrode and Accumet AR Dual Channel pH meter. TISAB II ionic strength adjuster was added in a 1:1 ratio with a sample volume (e.g. 5 ml), and standards were and measured in order to generate the calibration curve. Conversions from mV to ppm were determined based on the calibration curve and the data are presented in Table 7, 8 and 9.

**Table 7. 30-day fluoride stability data at 40°C.**

| | | | Concentration (in ppm ± standard deviation) of available fluoride [F⁻] after 30 days of accelerated aging (measured in triplicate): | | | | | |
|---|---|---|---|---|---|---|---|---|
| **Compound** | **MW (g/mol )** | **% Ca** | **5 ppm [Ca]** | **15 ppm [Ca]** | **25 ppm [Ca]** | **40 ppm [Ca]** | **50 ppm [Ca]** | **60 ppm [Ca]** |
| NaF | 42.0 | 0.0 | 221.2 (0.5) | | | | | |
| β-TCP | 310.2 | 38.7 | 209.8 (0.5) | 212.4 (0.5) | 210.7 (0.5) | 209.2 (0.9) | 213.3 (1.0) | 214.5 (0.0) |

**Table 8. 60-day fluoride stability data at 40°C.**

| | | | Concentration (in ppm ± standard deviation) of available fluoride [F⁻] after 60 days of accelerated aging (measured in triplicate): | | | | | |
|---|---|---|---|---|---|---|---|---|
| **Compound** | **MW (g/mol)** | **% Ca** | **5ppm [Ca]** | **15 ppm [Ca]** | **25 ppm [Ca]** | **40 ppm [Ca]** | **50 ppm [Ca]** | **60 ppm [Ca]** |
| NaF | 42.0 | 0.0 | 225.4 (0.9) | | | | | |
| β-TCP | 310.2 | 38.7 | 207.7 (0.0) | 208.2 (1.3) | 207.1 (1.3) | 209.1 (1.3) | 210.2 (0.9) | 209.1 (0.5) |

**Table 9. 90-day fluoride stability data at 40°C.**

| | | | Concentration (in ppm ± standard deviation) of available fluoride [F⁻] after 90 days of accelerated aging (measured in triplicate): | | | | | |
|---|---|---|---|---|---|---|---|---|
| **Compound** | **MW (g/mol )** | **% Ca** | **5 ppm [Ca]** | **15 ppm [Ca]** | **25 ppm [Ca]** | **40 ppm [Ca]** | **50 ppm [Ca]** | **60 ppm [Ca]** |
| NaF | 42.0 | 0.0 | 226.5 (0.9) | | | | | |
| β-TCP | 310.2 | 38.7 | 207.7 (0.5) | 207.2 (0.5) | 206.3 (1.0) | 206.6 (0.8) | 207.4 (0.0) | 205.2 (0.5) |

Acceptable fluoride levels should lie within 10% of the theoretical at 30, 60, and 90 days. In consideration of the full range evaluated (5 ppm Ca to 60 ppm Ca), β-TCP provided sufficient availability over the range of the tested calcium concentrations.

β-TCP at a level of 5 ppm calcium ion provided good stability of fluoride present at 225 ppm, representing a fluoride ion to calcium ion ratio of 45:1.

β-TCP at a level of 15 ppm calcium ion provided good stability of fluoride present at 225 ppm, representing a fluoride ion to calcium ion ratio of 15:1.

β-TCP at a level of 25 ppm calcium ion provided good stability of fluoride present at 225 ppm, representing a fluoride ion to calcium ion ratio of 9:1.

β-TCP at a level of 40 ppm calcium ion provided good stability of fluoride present at 225 ppm, representing a fluoride ion to calcium ion ratio of 5.625:1.

β-TCP at a level of 50 ppm calcium ion provided good stability of fluoride present at 225 ppm, representing a fluoride ion to calcium ion ratio of 4.5:1.

β-TCP at a level of 60 ppm calcium ion provided good stability of fluoride present at 225 ppm, representing a fluoride ion to calcium ion ratio of 3.75:1.

## Claims

1. A homogenous aqueous oral care composition comprising:
- a fluoride salt that directly provides free fluoride ions in the oral care composition, which provides the composition with from 10ppm to 5000ppm fluoride ions, and
- an unfunctionalised β-tricalcium phosphate,
wherein the fluoride salt and the β-tricalcium phosphate define a weight ratio of fluoride ions to total calcium ions, and wherein the weight ratio ranges in value from 2:1 to 100:1, such that the β-tricalcium phosphate portion is present in a catalytic and fluoride-stable amount relative to the fluoride salt portion,
wherein all ingredients of the oral care composition are combined in one mixture, such that they are not kept apart in separate compartments or by means of an anhydrous carrier.

2. The composition according to claim 1, wherein the fluoride salt is selected from the group consisting of alkali metal fluorides, stannous fluoride and combinations thereof.

3. The composition according to claim 2, wherein the fluoride salt is sodium fluoride.

4. The composition according to claim 1, wherein the weight ratio of fluoride ions to total calcium ions ranges from 2.5:1 to 50:1.

5. The composition according to claim 4, wherein the weight ratio of fluoride ions to total calcium ions ranges from 3:1 to 40:1.

6. The composition according to claim 5, wherein the weight ratio of fluoride ions to total calcium ions ranges from 3.5:1 to 30:1.

7. The composition according to claim 1, further comprising fluorapatite.

8. The composition according to claim 1, further comprising a desensitising agent.

9. The composition according to claim 8, wherein the desensitising agent is selected from the group consisting of a strontium salt, a potassium salt, and combinations thereof.

10. The composition according to claim 1, further comprising an anti-erosion agent.

11. The composition according to claim 1, further comprising a formulating agent selected from the group consisting of abrasives, surfactants, thickening agents, humectants, flavouring agents, sweetening agents, opacifying agents, colouring agents, preservatives and combinations thereof.

12. The composition according to claim 1, further comprising a matrix material, wherein the matrix material is selected from the group consisting of toothpastes, sprays, mouthwashes, gels, and combinations thereof.

## Patentansprüche

1. Homogene wässrige Mundpflegezusammensetzung umfassend:
- ein Fluoridsalz, das direkt in der Mundpflegezusammensetzung freie Fluoridionen bereitstellt, welches der Zusammensetzung 10 ppm bis 5000 ppm Fluoridionen bereitstellt, und
- ein unfunktionalisiertes β-Tricalciumphosphat,
wobei das Fluoridsalz und das β-Tricalciumphosphat ein Gewichtsverhältnis der Fluoridionen zu den gesamten Calciumionen definieren, und wobei das Gewichtsverhältnis in einem Wertebereich von 2:1 zu 100:1 liegt, so dass der β-Tricalciumphosphat-Anteil in einer katalytisch und fluorid-stabilen Menge relativ zu dem Fluoridsalz-Anteil vorhanden ist,
wobei alle Inhaltsstoffe der Mundpflegezusammensetzung in einer Mischung kombiniert sind, so dass sie nicht in getrennten Kompartimenten oder durch einen wasserfreien Träger auseinander gehalten sind.

2. Zusammensetzung nach Anspruch 1, wobei das Fluoridsalz aus der Gruppe bestehend aus Alkalimetallfluoriden, Zinnfluorid und Kombinationen daraus ausgewählt ist.

3. Zusammensetzung nach Anspruch 2, wobei das Fluoridsalz Natriumfluorid ist.

4. Zusammensetzung nach Anspruch 1, wobei das Gewichtsverhältnis der Fluoridionen zu den gesamten Calciumionen im Bereich von 2,5:1 zu 50:1 liegt.

5. Zusammensetzung nach Anspruch 4, wobei das Gewichtsverhältnis der Fluoridionen zu den gesamten Calciumionen im Bereich von 3:1 zu 40:1 liegt.

6. Zusammensetzung nach Anspruch 5, wobei das Gewichtsverhältnis der Fluoridionen zu den gesamten Calciumionen im Bereich von 3,5:1 zu 30:1 liegt.

7. Zusammensetzung nach Anspruch 1, weiter umfassend Fluorapatit.

8. Zusammensetzung nach Anspruch 1, weiter umfassend ein Desensibilisierungmittel.

9. Zusammensetzung nach Anspruch 8, wobei das Desensibilisierungmittel aus der Gruppe bestehend aus einem Strontiumsalz, einem Kaliumsalz und Kombinationen daraus ausgewählt ist.

10. Zusammensetzung nach Anspruch 1, weiter umfassend ein Anti-Erosionsmittel.

11. Zusammensetzung nach Anspruch 1, weiter umfassend ein Formulierungsmittel ausgewählt aus der Gruppe bestehend aus Schleifmitteln, Tensiden, Verdickungsmitteln, Feuchthaltemitteln, Aromastoffen, Süßstoffen, Trübungsmitteln, Farbstoffen, Konservierungsmitteln und Kombinationen daraus.

12. Zusammensetzung nach Anspruch 1, weiter umfassend ein Matrixmaterial, wobei das Matrixmaterial aus der Gruppe bestehend aus Zahnpasten, Sprays, Mundwässern, Gelen und Kombinationen daraus ausgewählt ist.

## Revendications

1. Composition de soin bucco-dentaire aqueuse homogène comprenant :
- un sel de fluorure qui fournit directement des ions fluorure libres dans la composition de soin bucco-dentaire, qui fournit à la composition de 10 ppm jusqu'à 5000 ppm d'ions fluorure, et
- un phosphate β-tricalcique non fonctionnalisé,
dans lequel le sel de fluorure et le phosphate β-tricalcique définissent un rapport en poids d'ions fluorure au total d'ions calcium, et dans lequel le rapport en poids varie en valeur de 2:1 jusqu'à 100:1, de sorte que la portion de phosphate β-tricalcique est présente en une quantité catalytique et stable au fluorure par rapport à la portion de sel de fluorure,
dans lequel tous les ingrédients de la composition de soin buccal sont combinés en un mélange, de telle sorte qu'ils ne soient pas séparés dans des compartiments séparés ou au moyen d'un support anhydre.

2. Composition selon la revendication 1, dans laquelle le sel de fluorure est choisi dans le groupe constitué par des fluorures de métal alcalin, du fluorure stanneux et combinaisons de ceux-ci.

3. Composition selon la revendication 2, dans laquelle le sel de fluorure est du fluorure de sodium.

4. Composition selon la revendication 1, dans laquelle le rapport en poids des ions fluorure au total des ions calcium varie de 2,5 : 1 jusqu'à 50 : 1.

5. Composition selon la revendication 4, dans laquelle le rapport en poids des ions fluorure au total des ions calcium varie de 3 : 1 jusqu'à 40 : 1.

6. Composition selon la revendication 5, dans laquelle le rapport en poids des ions fluorure au total des ions calcium varie de 3,5 : 1 jusqu'à 30 : 1.

7. Composition selon la revendication 1, comprenant en outre de la fluorapatite.

8. Composition selon la revendication 1, comprenant en outre un agent désensibilisant.

9. Composition selon la revendication 8, dans laquelle l'agent désensibilisant est choisi dans le groupe constitué par un sel de strontium, un sel de potassium et combinaisons de ceux-ci.

10. Composition selon la revendication 1, comprenant en outre un agent anti-érosion.

11. Composition selon la revendication 1, comprenant en outre un agent de formulation choisi dans le groupe constitué par les abrasifs, les tensioactifs, les agents épaississants, les humectants, les agents aromatisants, les édulcorants, les agents opacifiants, les agents colorants, les conservateurs et combinaisons de ceux-ci.

12. Composition selon la revendication 1, comprenant en outre un matériau matrice, le matériau matrice étant choisi dans le groupe constitué par les dentifrices, les sprays, les bains de bouche, les gels et combinaisons de ceux-ci.
